# FASCICULE DE BREVET EUROPEEN

(11) **EP 3 744 339 B1**
(45) Date de publication et mention de la délivrance du brevet: **22.12.2021**
(21) Numéro de dépôt: 19305672.8
(22) Date de dépôt: 28.05.2019
(51) Int. Cl.: A61K 36/752, A61Q 19/00, A61Q 19/08, A61K 8/9789

(54) **EXTRAIT FERMENTE DE PARTIES AERIENNES DE NEROLI**
FERMENTIERTER AUSZUG AUS DEN OBERIRDISCHEN TEILEN VON NEROLI
FERMENTED EXTRACT OF AERIAL PARTS OF NEROLI

(43) Date de publication de la demande: 02.12.2020
(73) Titulaire: Chanel Parfums Beauté, 92200 Neuilly-sur-Seine (FR)
(72) Inventeur: LEGANGNEUX, David, 93694 PANTIN CEDEX (FR); TORIBIO, Alix, 93694 PANTIN CEDEX (FR); LEJEUNE, François, 93694 PANTIN CEDEX (FR); BOUISSOU-CADIO, Emmanuelle, 93694 PANTIN CEDEX (FR); GENDRONNEAU, Gaëlle, 93694 PANTIN CEDEX (FR)
(74) Mandataire: Plasseraud IP

(56) Documents cités:
- CN-A- 102 743 312
- CN-A- 108 904 398
- FR-A1- 3 032 352
- KR-A- 20190 079 202
- TW-A- 201 010 738
- INESS JABRI KAROUI ET AL: "Characterization of Bioactive Compounds in Tunisian Bitter Orange ( Citrus aurantium L.) Peel and Juice and Determination of Their Antioxidant Activities", BIOMED RESEARCH INTERNATIONAL, vol. 2013, 1 janvier 2013 (2013-01-01), pages 1-12, XP055639971, ISSN: 2314-6133, DOI: 10.1155/2013/345415
- KIM SANG SUK ET AL: "Phytochemical, antioxidant, and antibacterial activities of fermentedCitrus unshiubyproduct", FOOD SCIENCE AND BIOTECHNOLOGY, THE KOREA SOC. OF FOOD SCIENCE AND TECHNOLOGY, HEIDELBERG, vol. 26, no. 2, 30 avril 2017 (2017-04-30) , pages 461-466, XP036215210, ISSN: 1226-7708, DOI: 10.1007/S10068-017-0063-9 [extrait le 2017-04-30]

## Description

### Domaine de l'invention

La présente invention a pour objet un extrait fermenté de parties aériennes de néroli (*Citrus aurantium*), son procédé d'obtention, une composition cosmétique ou dermatologique le contenant ainsi que différentes utilisations cosmétiques.

### Arrière-plan technique de l'invention

La peau est principalement constituée de trois couches, à savoir, en partant de la plus superficielle, l'épiderme, le derme et l'hypoderme.

L'épiderme est en particulier constitué de kératinocytes (majoritaires), de mélanocytes (intervenant dans la pigmentation de la peau) et de cellules de Langerhans. Sa fonction est de protéger le corps de l'environnement extérieur et d'assurer son intégrité, et notamment de freiner la pénétration de micro-organismes ou de substances chimiques, et d'empêcher l'évaporation de l'eau contenue dans la peau.

Pour ce faire, les kératinocytes subissent un processus de maturation orientée continu au cours duquel les kératinocytes situés dans la couche basale de l'épiderme forment, au stade terminal de leur différenciation, des cornéocytes qui sont des cellules mortes totalement kératinisées sous forme d'enveloppes cornées constituées de protéines et de lipides tels que des céramides. Lors de ce processus de différenciation, des lipides épidermiques intercornéocytaires sont en outre formés puis organisés sous forme de bicouches (feuillets) dans le *stratum corneum.* Ils participent, avec les enveloppes cornées précitées, à la fonction barrière de l'épiderme.

Lors du vieillissement cutané la peau s'affine, elle perd en volume et élasticité et sa fonction barrière s'en trouve altérée.

La production de radicaux libres est l'un des facteurs majeurs de l'accélération du vieillissement cutané. Cette production résulte majoritairement de l'exposition aux UV, de la pollution et des variations climatiques. Elle entraine un stress oxydatif qui est à l'origine de la dégradation de plusieurs éléments constitutifs de l'organisme, tels que les protéines, les acides gras, mais aussi l'ADN des cellules.

Ces radicaux libres peuvent être neutralisés par l'action d'antioxydants. L'un des moyens connus pour lutter contre le vieillissement cutané est d'appliquer sur la peau des compositions cosmétiques renfermant de tels antioxydants.

En raison d'une volonté toujours plus grande des consommateurs de se tourner vers des produits naturels renfermant le moins d'ingrédients synthétiques possibles, et au vu des contraintes réglementaires de plus en plus lourdes pesant sur les composés issus de l'industrie chimique, les antioxydants issus d'extraits végétaux sont aujourd'hui privilégiés.

Il existe un réel besoin pour de nouvelles molécules antioxydantes d'origine végétale qui permettent de lutter efficacement contre le vieillissement cutané et contre les méfaits du stress oxydatif de manière générale.

### Résumé de l'invention

La demanderesse a maintenant démontré qu'un extrait de parties aériennes de néroli (*Citrus aurantium*) présente des capacités antioxydantes remarquables. Cet extrait a pour particularité d'être un extrait fermenté, c'est-à-dire obtenu par un procédé d'extraction impliquant une étape de fermentation. Comme démontré dans la présente demande, un tel extrait permet de réduire le la teneur des cellules en radicaux libres en activant des voies de réponse au stress oxydatif, notamment la voie NRF2 (Nuclear factor erythroid-2-related factor 2).

Aussi dans un premier aspect, la présente invention porte sur un extrait de parties aériennes de *Citrus aurantium* fermenté.

Un tel extrait est susceptible d'être obtenu par un procédé d'extraction comprenant une étape de fermentation d'un extrait de parties aériennes de *Citrus aurantium* avec une levure de l'espèce *Saccharomyces cerevisiae.*

La présente invention porte donc également sur un extrait fermenté de parties aériennes de *Citrus aurantium* susceptible d'être obtenu par un procédé comprenant une étape de fermentation d'un extrait de parties aériennes de *Citrus aurantium* par une levure appartenant à l'espèce *Saccharomyces cerevisiae.*

L'invention a pour objet, selon un autre aspect, une composition cosmétique et/ou dermatologique comprenant, dans un milieu physiologiquement acceptable, au moins un extrait fermenté de *Citrus aurantium* tel que décrit précédemment.

Enfin, l'invention porte également sur l'utilisation de l'extrait décrit précédemment pour prévenir/réduire le vieillissement cutané, mais aussi sur son utilisation en tant qu'actif antioxydant, pour prévenir/réduire le stress oxydatif et/ou pour détoxifier la peau.

### Description détaillée de l'invention

Selon un premier aspect, la présente invention porte sur un extrait de parties aériennes de *Citrus aurantium* fermenté. Cet extrait est susceptible d'être obtenu par un procédé comprenant une étape de fermentation d'un extrait de *Citrus aurantium* par une levure appartenant à l'espèce *Saccharomyces cerevisiae.*

Le *Citrus aurantium* communément appelé « néroli » ou « Bigaradier » est une plante ligneuse de la famille des Rutaceae. C'est un arbre de 3 à 10 mètres, épineux, à feuilles persistantes, à fleurs odorantes et dont les fruits sont comestibles. L'Extrait selon la présente invention est un extrait obtenu à partir des parties aériennes du *Citrus aurantium,* c'est-à-dire un extrait obtenu à partir des fleurs, des feuilles des fruits ou des rameaux ou d'un mélange de ces derniers. Dans un mode de réalisation préféré, l'extrait est un extrait de fleurs de *Citrus aurantium,* également appelées « fleurs d'oranger ».

Par extrait « fermenté » on entend ici un extrait qui a subi une étape de fermentation. Dans le cadre de la présente invention, cette étape de fermentation est effectuée avec une levure appartenant à l'espèce *Saccharomyces cerevisiae.*

Les levures de l'espèce *Saccharomyces cerevisiae* sont connues et particulièrement utilisées dans l'industrie agroalimentaire, notamment dans le domaine de la panification ou pour la fermentation de boissons alcoolisées, par exemple dans le domaine de l'œnologie. L'homme du métier sait comment obtenir de telles levures pour mettre en œuvre la présente invention.

Dans un mode de réalisation particulier, ladite levure appartient à la variété *Saccharomyces cerevisiae* var. *bayanus.* Selon ce mode de réalisation, la levure peut être choisie parmi les souches de levures commercialisées sous les références IOC Fizz+, IOC Divine, IOC 18-2007 ou un mélange de celles-ci. Toutes ces souches sont référencées par l'institut français de la vigne et du vin et facilement accessibles pour l'homme du métier.

La souche de levures IOC Fizz+ est un mélange de deux levures déposées à l'Institut Pasteur sous les références LYCC 6022 : LA CLAIRE CGC62 et LYCC 6039 : LA CLAIRE SP665.

La souche IOC Divine correspond quant à elle à la souche déposée à l'Institut Pasteur sous la référence LYCC 7000, et la souche 18-2007 à celle déposée à l'Institut Pasteur sous la référence CNCM I-5320.

Les souches de levures utilisées dans le cadre de la présente invention peuvent être sous forme sèche, sous forme liquide ou sous forme de crème de levures.

Dans un mode de réalisation particulier, la souche de *Saccharomyces cerevisiae* est sous forme sèche.

Typiquement, l'étape de fermentation se fait en utilisant la souche de *Saccharomyces cerevisiae* à entre 1 et 5% en masse par rapport à la solution à fermenter, particulièrement à entre 2 et 3% en masse par rapport à la solution à fermenter, et plus particulièrement à 2% en masse par rapport à la solution à fermenter.

Dans un mode de réalisation particulier, l'étape de fermentation peut être effectuée au moyen d'un levain. Ce levain est préparé à partir d'un extrait de *Citrus aurantium* concentré fermenté avec la souche de *Saccharomyces cerevisiae.* L'extrait utilisé pour le levain est typiquement concentré jusqu'à obtenir un taux de matière sèche de l'ordre de 5 à 30%.

Selon ce mode de réalisation, l'étape de fermentation est effectuée en ajoutant ce levain à l'extrait de *Citrus aurantium.* L'utilisation d'un levain permet d'amorcer la fermentation et d'améliorer ainsi la cinétique de l'étape de fermentation.

Typiquement, le levain est préparé en fermentant pendant environ 2 heures à température ambiante (ou alternativement à (ou alternativement à 37°C) un extrait de *Citrus aurantium* concentré à entre 5 et 50%, particulièrement à entre 15 et 30% de matière sèche dans l'eau avec 10% en poids de levures sèches *Saccharomyces cerevisiae.* 1 à 5% de ce levain en poids, préférentiellement 2% de levain en poids, sont ajoutés à l'extrait de *Citrus aurantium* pour l'étape de fermentation.

L'extrait de *Citrus aurantium* utilisé pour obtenir l'extrait fermenté selon l'invention peut-être obtenu selon n'importe quel procédé d'extraction connu de l'homme du métier.

Typiquement, un procédé de préparation d'un extrait végétal comprend les étapes suivantes:
a) l'extraction de la plante (ici des parties aériennes de *Citrus aurantium*) avec au moins un solvant alcoolique et/ou de l'eau ;
b) la filtration, par exemple par tamisage, du mélange obtenu en a) afin d'éliminer les résidus végétaux,
c) optionnellement, la décoloration du mélange obtenu à l'issue l'étape b; et
d) l'élimination du solvant et la concentration de l'extrait.

Afin d'obtenir l'Extrait fermenté de *Citrus aurantium* selon la présente invention, on ajoutera typiquement une étape e) après les étapes a) à d) ci-dessus, cette étape e) comprenant la fermentation de l'extrait obtenu à l'issu de l'étape d) avec une levure appartenant à l'espèce *Saccharomyces cerevisiae.*

L'étape a) peut en outre comprendre le broyage de la plante, typiquement, de ses fleurs et/ou de ses feuilles, afin d'obtenir des particules de taille inférieure à 5 cm, de préférence inférieure à 2 cm.

L'étape d'extraction est effectuée avec au moins d'un solvant d'extraction constitué d'un solvant alcoolique et/ou de l'eau. Le ratio plante/solvant typiquement de 1 pour 10 (poids/poids).

Dans un mode de réalisation particulier, le solvant alcoolique est de l'éthanol à 96°.

Le solvant d'extraction peut contenir de 0 à 100% en volume d'eau et de 0 à 100% en volume de solvant alcoolique. Dans un mode de réalisation particulier, le solvant d'extraction comprend 100% d'eau, dans un autre mode de réalisation, il comprend 100% d'éthanol. Le solvant d'extraction peut également comprendre 50% en volume d'eau et 50% en volume d'éthanol..

Dans un mode de réalisation particulier, l'eau peut être de l'eau déionisée. Dans le cadre de la présente invention, le solvant d'extraction est typiquement de l'eau déionisée.

L'étape d'extraction dure au moins 1 heure, de préférence au moins 2 heures, particulièrement au moins 3 heures, et peut être renouvelée une à deux fois.

Comme indiqué ci-dessus, le procédé d'extraction peut comprendre une étape dans laquelle le mélange issu de l'étape b) est décoloré. Cette étape vise à éliminer les pigments présents dans l'extrait tels que les chlorophylles et les xanthophylles. L'homme du métier connait plusieurs méthodes permettant d'éliminer ces pigments. La décoloration peut, par exemple, être effectuée en mettant le mélange en contact avec du charbon actif. Une fois la décoloration effectuée, le mélange est filtré afin d'éliminer les résidus de charbon.

Typiquement, l'étape d) comprend la concentration de l'extrait jusqu'à obtenir un taux de matière sèche dans l'eau de l'ordre 5 à 30%, de manière préférée de 15% à 30%.

Cette étape de concentration est typiquement effectuée par évaporation du solvant d'extraction (solvant alcoolique et/ou eau). Cette extraction peut par exemple être faite sous vide.

L'extrait de *Citrus aurantium* issu de l'étape d, c'est-à-dire l'extrait de *Citrus aurantium* concentré, peut être utilisé pour préparer le levain mentionné plus haut.

Le procédé décrit ci-dessus peut en outre comprendre une étape d') entre les étapes d) et e), dans laquelle le mélange contenant l'extrait concentré est finement filtré (typiquement à 2µm, voire 0.2µm) afin d'éliminer les particules fines et les bactéries résiduelles.

Le procédé peut comprendre en outre une étape f), dans laquelle l'extrait fermenté est filtré puis dilué afin d'obtenir un extrait contenant 1 à 15% de matière sèche en poids, particulièrement de l'ordre de 10% de matière sèche en poids. Cet extrait peut se présenter sous forme de solution aqueuse limpide et stable.

Dans un mode de réalisation particulier, le procédé permettant d'obtenir l'extrait fermenté de *Citrus aurantium* selon la présente invention comprend les étapes suivantes :
a) Une poudre de fleurs d'oranger broyées à une finesse inférieure à 2cm est extraite deux fois avec de l'eau désionisée à 75 °C, durant 2 heures minimum, le ratio plante/solvant est de 1 pour 10 (poids/poids) ;
b) Le mélange est filtré jusqu'à 4µm ;
c) Le mélange résultant est soumis à une décoloration par contact avec du charbon activé pendant une heure puis subit une microfiltration jusqu'à 1µm afin d'éliminer les résidus de charbon ;
d) L'extrait est concentré par élimination d'une partie de l'eau par évaporation sous vide jusqu'à atteindre un taux de matière sèche proche de 15% (poids/poids) ;
d') Le mélange est filtré à 0.2µm ;
e) Fermentation pendant 24 à 48 heures à 37°C du mélange avec 2% en poids d'un levain, ledit levain ayant été obtenu en concentrant une partie de l'extrait obtenu avant l'étape d) à 30% de matière sèche dans l'eau et en y ajoutant 10% en poids de levures sèches *Saccharomyces cerevisiae* pour une première fermentation durant 2 heures à 37°C ;
f) L'extrait fermenté est ensuite filtré à 1µm, puis dilué pour obtenir un extrait contenant 1 à 10% de matière sèche en poids auquel est ajouté 0.7% de phénoxyéthanol ou 20% de 1,3-propanediol (10% d'extrait sec/70% d'eau, ratio en poids/poids) pour une meilleure conservation.

L'Extrait fermenté selon la présente invention présente des propriétés antioxydantes remarquables. Comme démontré dans les exemples ci-dessous (voir particulièrement exemples 2 et 3), il permet de réduire significativement le stress oxydatif dans des cellules de peau telles que des kératinocytes. Il permet notamment d'activer la voie NRF2 (Nuclear factor erythroid-2-related factor 2) qui est connue pour être une voie de réponse au stress oxydatif. NRF2 est un facteur de transcription qui, de par sa liaison à la séquence régulatrice appelée élément de réponse antioxydant (ARE), va permettre l'expression de gènes codant pour des enzymes antioxydantes. L'activation de cette voie a donc pour effet de réduire la teneur des tissus en radicaux libres grâce à l'action d'agents antioxydants.

Comme expliqué ci-dessus, la production de radicaux libres et le stress oxydatif que cette production engendre, est l'un des facteurs majeurs de l'accélération du vieillissement cutané. La diminution des radicaux libres, et donc du stress oxydatif, permettent de lutter contre les signes du vieillissement cutané tels que les rides et ridules, la perte de fermeté et d'élasticité dues à une perte tissulaire au niveau de l'épiderme et/ou du derme ; la perte d'éclat due à la réduction de la microcirculation et à un ralentissement du renouvellement cellulaire au niveau de l'épiderme, l'apparition de taches pigmentaires associées à un dysfonctionnement de la synthèse de la mélanine, ou encore la sécheresse cutanée résultant d'une diminution de la fonction barrière de la couche cornée et à un ralentissement du renouvellement épidermique.

Aussi selon un autre aspect, la présente invention porte sur l'utilisation d'un extrait fermenté de parties aériennes de *Citrus aurantium* pour prévenir/réduire le vieillissement cutané.

L'invention porte également sur l'utilisation d'un extrait fermenté de parties aériennes de *Citrus aurantium* en tant qu'actif antioxydant et/ou pour prévenir/réduire le stress oxydatif et/ou pour détoxifier la peau.

Un actif « antioxydant » est un composé ayant des propriétés antiradicalaires, c'est-à-dire ayant la capacité de neutraliser les radicaux libres, et notamment les réactions d'oxydation liées à ces radicaux libres. Cette neutralisation entraine une diminution de la teneur en radicaux libres dans les tissus.

Le « stress oxydatif » est un déséquilibre entre la quantité excessive de radicaux libres et des antioxydants dans les tissus. La neutralisation des radicaux libres par l'apport en actifs antioxydants permet donc de réduire ce stress oxydatif. L'exemple 3 ci-dessous montre un moyen de mesurer le stress oxydatif et notamment l'effet d'un actif antioxydant sur ce stress oxydatif.

La diminution des radicaux libres et du stress oxydatif dans la peau permet de « détoxifier » la peau, c'est-à-dire d'obtenir un effet « detoxifiant ». On entend par « détoxifiant » un effet permettant de lutter contre les effets néfastes des radicaux libres sur la peau.

L'extrait selon la présente invention peut avantageusement être intégré dans une composition cosmétique ou dermatologique. Aussi, selon un autre aspect, la présente invention porte sur une composition cosmétique ou dermatologique comprenant, dans un véhicule cosmétiquement ou pharmaceutiquement acceptable, un extrait alcoolique de parties *Citrus aurantium* selon l'invention.

De préférence, ledit extrait est présent dans la composition cosmétique ou dermatologique à raison de 0,001 à 10% en poids total de la composition, en particulier à raison de 0,01 à 10%, de préférence de 0,1 à 10% en poids total de la composition. Ladite composition cosmétique ou dermatologique peut notamment, être adaptée à une application par voie topique.

Avantageusement, ladite composition cosmétique ou dermatologique peut se présenter sous la forme d'une poudre, d'une émulsion, d'une microémulsion, d'une nanoémulsion, d'une suspension, d'une solution d'une lotion, d'une crème, d'un gel aqueux ou hydroalcoolique, d'une mousse, d'un sérum, d'une solution ou d'une dispersion pour aérosol, ou d'une dispersion de vésicules lipidiques.

Dans le cas d'une émulsion, il peut s'agir d'une émulsion eau dans huile ou huile dans eau.

La composition cosmétique ou dermatologique selon l'invention peut comprendre également un solvant choisi en fonction des différents ingrédients et de la forme d'administration.

A titre d'exemples, on peut citer l'eau (de préférence de l'eau déminéralisée), un alcool tel que l'éthanol, ou un éther de diéthylène glycol tel que l'éthoxydiglycol ou l'éther monométhylique de diéthylène glycol.

Ladite composition cosmétique peut également comprendre, en outre de l'extrait selon l'invention, au moins un additif usuel dans le domaine, tel que par exemple au moins un composé choisi parmi un agent émollient ou humectant, un agent gélifiant et/ou épaississant, un agent tensioactif, une huile, un agent actif, un colorant, un conservateur, un agent antioxydant, un agent actif, une poudre organique ou inorganique, un filtre solaire et un parfum.

Notamment, ladite composition peut contenir :
U n ou plusieurs agent(s) émollient(s) ou humectant(s), qui peuvent être choisi(s) par exemple parmi la glycérine, les glycols, les silicones hydrosolubles tels que celui vendu sous la dénomination KF6011 (Shin Etsu) et le Jojoba hydrosoluble, tel que celui vendu sous la dénomination Resplanta jojoba (Res pharma).

Ledit agent émollient ou humectant peut être présent dans la composition à une teneur de l'ordre de 0 à 30%, de préférence 2 à 10% en poids, par rapport au poids total de la composition.
- Un ou plusieurs agent(s) gélifiants(s) et/ou épaississant(s) de la phase aqueuse, choisi par exemple parmi les dérivés cellulosiques, gommes d'origine végétale (guar, caroube, alginates, carraghénanes, pectine), d'origine microbienne (xanthane), les argiles (laponite), les matériaux identifiés par les noms INCI "ammonium acryloyldiméthyltaurate/vp copolymer" et "ammonium acryloyldiméthyl-taurate/beheneth-25 méthacrylate copolymer" (tels que par exemple ceux vendus sous les dénominations Aristoflex AVC et HMB par Clariant).

Ledit agent gélifiant et/ou épaississant peut être présent dans la composition à une teneur de l'ordre de 0 à 10% en poids, par rapport au poids total de la composition.
U n ou plusieurs agent(s) tensioactif(s), de préférence non ionique, présent dans une teneur de l'ordre de 0 à 8%, de préférence 0,5 à 3% en poids, par rapport au poids total de la composition.
U n ou plusieurs corps gras liquide(s) à température ambiante, communément dénommé(s) huiles(s), volatil(s) ou non volatile(s), hydrocarboné(s) ou siliconé(s), linéaire (s), cyclique(s) ou ramifié (s), par exemple, l'isododécane, le cyclopentadimethylsiloxane, les diméthicones, l'isononanoate d'isononyle ou le pentaerythrityl tetraisostéarate, de préférence à raison de 0 à environ 10%, de préférence 0,5 à 5% en poids, par rapport au poids total de la composition.
U n ou plusieurs agent(s) actif(s), d'origine naturelle ou synthétique, ayant une activité biologique, par exemple choisis parmi les vitamines, les oligo-éléments, l'allantoïne, les protéines végétales, les extraits végétaux, les agents hydratants, les agents antiâges, les antioxydants, les agents favorisant l'éclat et des mélanges de ceux-ci. En particulier, l'agent actif est choisi parmi une eau de fruit de vanilla planifolia, le niacinamide, l'acide hyaluronique et ses dérivés, un extrait de levure et des mélanges de ceux-ci.
U n ou plusieurs colorant(s) hydrosoluble(s) tels que, par exemple, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine ou la xanthophylle, de préférence à raison de 0 à environ 2% en poids, par rapport au poids total de la composition.

D'autres additifs habituellement utilisés en cosmétique peuvent également être présents dans la composition selon l'invention, notamment des conservateurs, des agents antioxydants ou des parfums bien connus dans le domaine technique.

L'homme du métier est en mesure de choisir, parmi l'ensemble de ces éventuels additifs, aussi bien la nature que la quantité de ceux qui seront ajoutés à la composition, de telle sorte que celle-ci conserve l'ensemble de ses propriétés.

L'invention sera maintenant illustrée par les exemples non limitatifs suivants.

### BREVE DESCRIPTION DES FIGURES

**Figure 1** : Diagramme montrant les différentes voies biologiques modulées dans des kératinocytes par un extrait de néroli selon la présente invention.
**Figure 2****:** Diminution du stress oxydatif dans des kératinocytes après traitement avec un extrait de Néroli selon la présente invention.

### EXEMPLES

**EXEMPLE 1** - Procédé d'extraction du Néroli selon la présente invention:
1) Les fleurs séchées de fleurs d'oranger sont broyées à une finesse inférieure à 2cm ;
2) La poudre de fleurs d'oranger est ensuite extraite deux fois avec de l'eau désionisée à 75 °C, durant 2 heures minimum, le ratio plante/solvant est de 1 pour 10 (poids/poids) ;
3) Le mélange est tamisé à 100µm afin d'éliminer les résidus végétaux puis laissé au repos une nuit et à nouveau filtré jusqu'à 4µm ;
4) Le mélange résultant est soumis à une décoloration par contact avec du charbon activé pendant une heure afin d'éliminer les pigments tels que les chlorophylles et xanthophylles ;
5) Le mélange décoloré est séparé du résidu de charbon à l'aide d'une microfiltration (jusqu'à 1µm) ;
6) L'extrait est concentré par élimination d'une partie de l'eau par évaporation sous vide jusqu'à atteindre un taux de matière sèche proche de 15% (poids/poids) ;
7) Le mélange est filtré à 0.2µm pour éliminer les particules fines et les bactéries résiduelles ;
8) Afin de préparer le levain servant à la fermentation de l'extrait, une partie de l'extrait obtenu avant l'étape 6) est concentrée à 30% de matière sèche dans l'eau puis 10% en poids de levures sèches *Saccharomyces cerevisiae var. bayanus* y sont ajoutés. Le mélange est agité durant 2 heures à température ambiante. Ce mélange constitue le levain.
9) 2% de levain en poids sont ajoutés au mélange issu de l'étape 6) ;
10) Après 24 à 48 heures de fermentation à température ambiante sous agitation et dans l'obscurité, l'extrait fermenté est laissé à décanter une nuit pour éliminer les bulles de gaz formées ;
11) L'extrait fermenté est ensuite filtré à 1µm, puis dilué pour obtenir un extrait contenant 10% de matière sèche, 0.7% de phénoxyéthanol sont ajoutés pour une meilleure conservation ou 20% de 1,3-propanediol (10% d'extrait sec/70% d'eau, ratio en poids/poids) et le tout est filtré à 0.2µm.

Les teneurs de l'extrait des fleurs d'oranger sont données dans le tableau 1 ci-dessous.

**Tableau 1 : Composition d'un extrait de Néroli obtenu selon le procédé de l'invention**

| Famille moléculaire | Molécules | Extrait conventionnel | Extrait fermenté |
|---|---|---|---|
| Saccharides | Fructose, Glucose, Sucrose | 2,20% | 0,00% |
| Acides organiques et autres | Synephrine, Stachydrine, Acide quinique, Acide malique | 1,24% | 1,32% |
| Flavonoïdes | Neoriocitrine, Narirutine, Naringine, Hesperetine glucoside, Hesperidine, Neohesperidine | 2,43% | 4,40% |
| Autres | Autres | 4,13% | 4,64% |

Comme démontré ci-dessus, le procédé selon la présente invention permet d'éliminer complètement les sucres simples d'un extrait de fleurs d'oranger. Le procédé selon l'invention permet également d'augmenter la teneur en polyphénols de l'extrait.

### EXEMPLE 2 - Activation de la voie de réponse au stress oxydatif dans des kératinocytes humains normaux traités avec l'extrait de Néroli selon la présente invention.

### Protocole:

Des kératinocytes épidermiques humains normaux issus de trois donneurs différents (19 à 30 ans) ont été ensemencés en plaque 24 puits et cultivés en milieu complémenté kératinocyte-SFM pendant 48hrs à 37°C, 5% CO₂. Les cellules ont ensuite été incubées ou non (témoin) avec 0.4% d'extrait de Néroli pendant 24hrs. Les conditions ont été réalisées en n=2. A la fin de l'incubation, les surnageants de culture ont été éliminés et les tapis cellulaires rincés en PBS. Les ARNs totaux ont été extraits à l'aide de Tripure^{®} Isolation Reagent selon les recommandations du fournisseur. La quantité et la qualité des ARN ont été évaluées par électrophorèse capillaire (Bioanalyseur 2100, Agilent). Les ADN complémentaires ont été synthétisés et un transcriptome a été réalisé sur puce Affymetrix GeneChip Human Transcriptome Array 2.0. L'analyse bioinformatique des gènes dont l'expression est modulée par un facteur 2 minimum a été effectuée avec le logiciel Ingenuity Pathway Analysis (IPA^{®}, Qiagen). Ce logiciel recueille des informations sur les interactions molécule à molécule, les réseaux biologiques et les voies canoniques dans la base de données Ingenuity Knowledge.

### Résultats:

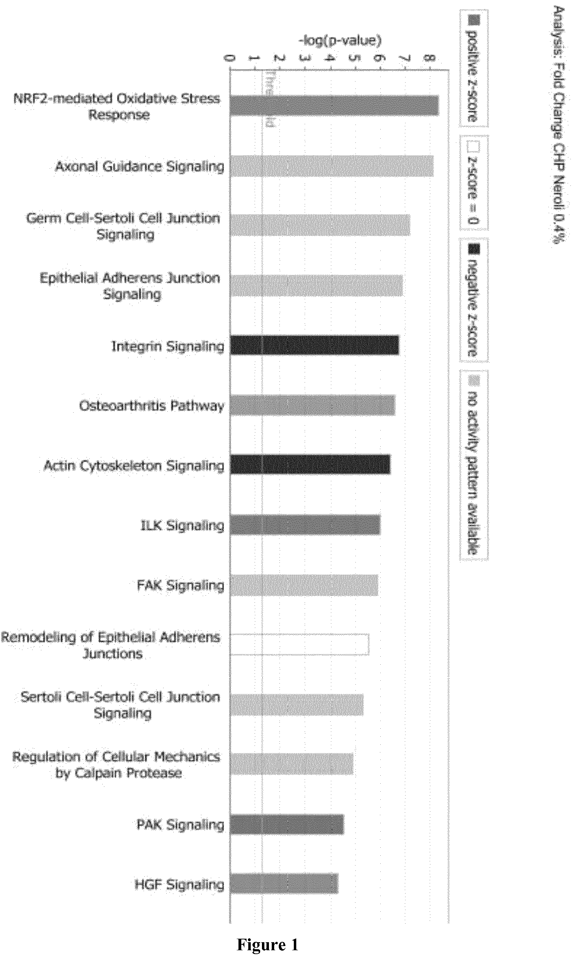

Les voies biologiques modulées par l'extrait de Néroli sont représentées dans la figure 1. La fonction cellulaire majeure qui est activée par l'extrait de Néroli dans les kératinocytes est la voie NRF2 de réponse au stress oxydatif avec 31 gènes impliqués. La liste des gènes surexprimés dans cette voie biologique est présentée dans le tableau 1.

| ^{©} 2000-2018 QIAGEN. All rights reserved. | | |
|---|---|---|
| **Symbol** | **Entrez Gene Name** | **Expr Fold Change** |
| GCLC | glutamate-cysteine ligase catalytic subunit | 8,330 |
| GPX2 | glutathione peroxidase 2 | 5,240 |
| MGST1 | microsomal glutathione S-transferase 1 | 5,210 |
| NQO1 | NAD(P)H quinone dehydrogenase 1 | 5,120 |
| ABCC2 | ATP binding cassette subfamily C member 2 | 4,510 |
| PIK3C2B | phosphatidylinositol-4-phosphate 3-kinase catalytic subunit type 2 beta | 4,140 |
| GSR | glutathione-disulfide reductase | 3, 690 |
| ABCC1 | ATP binding cassette subfamily C member 1 | 3,210 |
| CBR1 | carbonyl reductase 1 | 3,030 |
| TXNRD1 | thioredoxin reductase 1 | 2,840 |
| GSTM3 | glutathione S-transferase mu 3 | 2,830 |
| GSTA4 | glutathione S-transferase alpha 4 | 2,800 |
| FTH1 | ferritin heavy chain 1 | 2,780 |
| SOD2 | superoxide dismutase 2 | 2,750 |
| DNAJB1 | DnaJ heat shock protein family (Hsp40) member B1 | 2,590 |
| EPHX1 | epoxide hydrolase 1 | 2,490 |
| GSTM4 | glutathione S-transferase mu 4 | 2,400 |
| DNAJC3 | DnaJ heat shock protein family (Hsp40) member C3 | 2,380 |
| HSPB8 | heat shock protein family B (small) member 8 | 2,370 |
| GCLM | glutamate-cysteine ligase modifier subunit | 2,270 |
| PRDX1 | peroxiredoxin 1 | 2,230 |
| FKBP5 | FK506 binding protein 5 | 2,200 |
| FTL | ferritin light chain | 2,190 |
| TXN | thioredoxin | 2,070 |

### EXEMPLE 3 : Réduction du stress oxydatif dans des kératinocytes humains normaux traités avec l'extrait de Néroli selon la présente invention

### Protocole:

Des kératinocytes épidermiques humains normaux issus d'un donneur âgé de 27 ans ont été ensemencés en plaque 6 puits et cultivés pendant 72hrs à 37°C, 5% CO₂. Les cellules ont ensuite été incubées ou non (témoin) avec 0.4% d'extrait de Néroli pendant 24hrs avant de réaliser un stress oxydatif par traitement des cellules avec 50µM de ménadione sodium bisulfite (Sigma). Le stress oxydatif est visualisé à l'aide d'une sonde fluorescente Mitosox Red mitochondrial superoxide indicator (Life technologies). Des photographies des cultures ont été prises 1h30 après le stress et l'intensité du signal fluorescent a été quantifiée.

### Résultats:

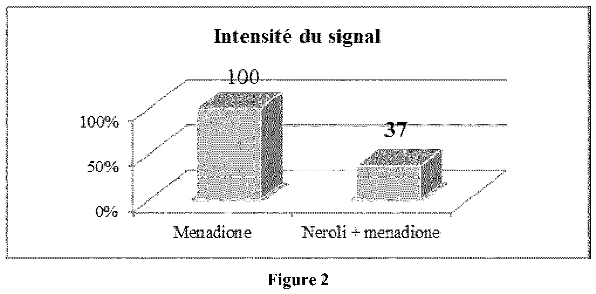

Une diminution de 63% de l'intensité du signal est observée après traitement avec l'extrait de Néroli comparativement à la condition témoin (figure 2).

### EXEMPLE 4 : Composition cosmétique

Les compositions suivantes peuvent être préparées de façon classique pour l'homme du métier. Les quantités indiquées ci-dessous sont exprimées en pourcentages pondéraux. Les ingrédients en majuscules sont identifiés conformément à la dénomination INCI.

### 4A - émulsion huile/eau

| **Nom INCI** | **(% W/W)** |
|---|---|
| Jojoba esters | 1-10 |
| limnanthes alba (meadowfoam) seed oil | 1-10 |
| undecane & tridecane & tocopherol & helianthus annuus (sunflower) seed oil | 1-10 |
| Butyrospermum parkii butter (LIPEX SHEASOFT) | 1-10 |
| Camellia kissi seed oil | 1-10 |
| Butyrospernum parkii butter extract (LIPEX SHEA TRIS) | 1-10 |
| Cetyl ethylhexanoate | 1-5 |
| Diisostearyl dimer dilinoleate (SCHERCEMOL DISD) | 1-10 |
| sodium acrylates copolymer & lecithin | 0.1-5 |
| amodimethicone | 0.1-2 |
| Glyceryl stearate & PEG-100 stearate | 0.1-5 |
| CARBOMER | 0.01-5 |
| Alcaligenes polysaccharides | 1-10 |
| Silica | 0.1-10 |
| Sodium hyaluronate | 0.01-3 |
| Glycerin | 1-30 |
| Polyquaternium-51 | 1-10 |
| Adenosine | 0.1-0.5 |
| Niacinamide | 0.1-5 |
| Palmitoyl Tripeptide-1 & Palmitoyl Tetrapeptide-7 | 1-5 |
| Secale Cereale (Rye) Seed Extract | 1-5 |
| Extrait selon l'invention | 0.001-10 |
| Ascorbyl glucoside | 0.001-5 |
| Glycols (Caprylyl Glycol and/or Pentylene Glycol and/or Butylene Glycol and/or propanediol) | 0,1-10 |
| Water | Qs 100 |

### 4b - émulsion huile/eau crème

| **Nom INCI** | **(% w/w)** |
|---|---|
| Behenyl alcohol | 1-5 |
| Cetyl alcohol | 0.1-5 |
| Phenyl trimethicone | 1-5 |
| Dimethicone & Dimethicone/Vinyl Dimethicone Crosspolymer | 1-30 |
| Ectoin | 0.1-5 |
| PPG-2 myristyl ether propionate | 1-10 |
| Titanium Dioxide | 1-20 |
| Zinc Dioxide | 1-20 |
| Diethylamino Hydroxybenzoyl Hexyl Benzoate (Uvinul A+) | 1-5 |
| Bis-Ethylhexyloxyphenol Methoxyphenyl Triazine (Tinosorb S) | 1-5 |
| (Tinosorb M) | 1-5 |
| Ethyl hexyl Methoxycinnamate | 1-7.5 |
| Polysilicone -11 | 1-5 |
| Silica | 1-5 |
| lauroyl lysine | 1-5 |
| C20-22 alkyl phosphate & C20-22 alcohols | 0.5-5 |
| Glyceryl stearate & PEG-100 stearate | 0.5-5 |
| sodium acrylate / sodium acryloyldimethyltaurate copolymer | 0.1-5 |
| Hydrogenated starch hydrolysate & maltooligosyl glucoside | 0.1-10 |
| Xanthan Gum | 0, 01-2 |
| Agar | 0.1-5 |
| Adenosine | 0.1-0.5 |
| Niacinamide | 0.1-5 |
| secale cereale (rye) seed extract | 0.1-5 |
| palmitoyl tetrapeptide-7 | 1-5 |
| vanilla planifolia fruit water | 0.1-2 |
| vanilla planifolia flower extract | 0.1-2 |
| yeast extract | 1-3 |
| Saccharide isomerate | 1-5 |
| Extrait selon l'invention | 0.001-10 |
| Licorice extract | 0.001-5 |
| Water | Qs 100 |

**Ces compositions peuvent être appliquées tous les jours, matin et/ou soir, sur la peau.**

## Revendications

1. Extrait de parties aériennes de *Citrus aurantium,* dans lequel ledit extrait étant un extrait fermenté.

2. Extrait fermenté de parties aériennes de *Citrus aurantium* susceptible d'être obtenu par un procédé comprenant une étape de fermentation d'un extrait de parties aériennes de *Citrus aurantium* par une levure appartenant à l'espèce *Saccharomyces cerevisiae.*

3. Extrait selon la revendication 2, dans lequel ladite levure de l'espèce *Saccharomyces cerevisiae* est de la variété *Saccharomyces cerevisiae* var. *bayanus.*

4. Extrait selon la revendication 3, dans lequel ladite levure de variété *Saccharomyces cerevisiae* var. *bayanus* est une levure de la souche IOC Fizz+.

5. Extrait selon la revendication 3, dans lequel ladite levure de variété *Saccharomyces cerevisiae* var. *bayanus* est une levure de la souche IOC Divine.

6. Extrait selon la revendication 3, dans lequel ladite levure de variété *Saccharomyces cerevisiae* var. *bayanus* est une levure de la souche IOC 18-2007.

7. Extrait selon l'une quelconque des revendications 1 à 6, dans lequel ledit extrait de *Citrus aurantium* est un extrait de fleurs.

8. Procédé d'obtention d'un extrait fermenté de parties aériennes de *Citrus aurantium,* ledit procédé comprenant les étapes suivantes :
a) l'extraction de parties aériennes de *Citrus aurantium* avec au moins un solvant alcoolique et/ou de l'eau ;
b) la filtration du mélange obtenu en a) afin d'éliminer les résidus végétaux;
c) optionnellement, la décoloration du mélange obtenu à l'issue l'étape b;
d) l'élimination du solvant d'extraction et la concentration de l'extrait obtenu ; et
e) fermentation de l'extrait obtenu de *Citrus aurantium* par une levure appartenant à l'espèce *Saccharomyces cerevisiae.*

9. Procédé selon la revendication 8, dans lequel l'étape de fermentation est effectuée au moyen d'un levain préparé à partir de la fermentation d'un extrait de *Citrus aurantium* concentré avec la souche de *Saccharomyces cerevisiae.*

10. Composition cosmétique comprenant un extrait de *Citrus aurantium* selon l'une des revendications 1 à 7.

11. Utilisation cosmétique de l'extrait selon l'une des revendications 1 à 7 pour prévenir/réduire le vieillissement cutané.

12. Utilisation cosmétique de l'extrait selon l'une des revendications 1 à 7 en tant qu'actif antioxydant et/ou pour prévenir/réduire le stress oxydatif et/ou pour détoxifier la peau.

## Patentansprüche

1. Extrakt aus oberirdischen Teilen von *Citrus aurantium,* wobei der Extrakt ein fermentierter Extrakt ist.

2. Fermentierter Extrakt aus oberirdischen Teilen von *Citrus aurantium,* welcher durch ein Verfahren erhalten werden kann, das einen Schritt des Fermentierens eines Extrakts aus oberirdischen Teieln von *Citrus aurantium* durch eine Hefe, die zur Spezies *Saccharomyces cerevisiae* gehört, umfasst.

3. Extrakt nach Anspruch 2, wobei die Hefe der Spezies *Saccharomyces cerevisiae* von der Sorte *Saccharomyces cerevisiae* var. *bayanus* ist.

4. Extrakt nach Anspruch 3, wobei die Hefe der Sorte *Saccharomyces cerevisiae* var. *bayanus* eine Hefe des Stammes IOC Fizz+ ist.

5. Extrakt nach Anspruch 3, wobei die Hefe der Sorte *Saccharomyces cerevisiae* var. *bayanus* eine Hefe des Stammes IOC Divine ist.

6. Extrakt nach Anspruch 3, wobei die Hefe der Sorte *Saccharomyces cerevisiae* var. *bayanus* eine Hefe des Stammes IOC 18-2007 ist.

7. Extrakt nach einem der Ansprüche 1 bis 6, wobei der *Citrus aurantium*-Extrakt ein Blütenextrakt ist.

8. Verfahren zur Gewinnung eines fermentierten Extrakts aus oberirdischen Teilen von *Citrus aurantium,* wobei das Verfahren die folgenden Schritte umfasst:
a) Extraktion von oberirdischen Teilen von *Citrus aurantium* mit wenigstens einem alkoholischen Lösungsmittel und/oder Wasser;
b) Filtrieren der in a) erhaltenen Mischung, um Pflanzenreste zu entfernen;
c) gegebenenfalls Entfärbung der in Schritt b erhaltenen Mischung;
d) Entfernen des Extraktionslösungsmittels und Konzentrieren des erhaltenen Extrakts; und
e) Fermentation des aus *Citrus aurantium* gewonnenen Extrakts mittels einer Hefe der Spezies *Saccharomyces cerevisiae.*

9. Verfahren nach Anspruch 8, wobei der Fermentationsschritt mittels einer Vorkultur durchgeführt wird, die durch die Fermentation eines konzentrierten Extrakts von Ci*trus aurantium* mit dem Stamm *Saccharomyces cerevisiae* hergestellt wurde.

10. Kosmetische Zusammensetzung, umfassend einen *Citrus aurantium*-Extrakt nach einem der Ansprüche 1 bis 7.

11. Kosmetische Verwendung des Extrakts nach einem der Ansprüche 1 bis 7 zur Vorbeugung/Verringerung der Hautalterung.

12. Kosmetische Verwendung des Extrakts nach einem der Ansprüche 1 bis 7 als antioxidativer Wirkstoff und/oder zur Vorbeugung/Verminderung von oxidativem Stress und/oder zur Entgiftung der Haut.

## Claims

1. Extract of aerial parts of *Citrus aurantium,* wherein said extract is a fermented extract.

2. Fermented extract of aerial parts of *Citrus aurantium* obtainable by a process comprising a step of fermentation of an extract of aerial parts of *Citrus aurantium* with a yeast belonging to the species *Saccharomyces cerevisiae.*

3. Extract according to Claim 2, wherein said yeast of the species *Saccharomyces cerevisiae* is of the variety *Saccharomyces cerevisiae* var. *bayanus.*

4. Extract according to Claim 3, wherein said yeast of the variety *Saccharomyces cerevisiae* var. *bayanus* is a yeast of the IOC Fizz+ strain.

5. Extract according to Claim 3, wherein said yeast of the variety *Saccharomyces cerevisiae* var. *bayanus* is a yeast of the IOC Divine strain.

6. Extract according to Claim 3, wherein said yeast of the variety *Saccharomyces cerevisiae* var. *bayanus* is a yeast of IOC 18-2007 strain.

7. Extract according to any one of claims 1 to 6, wherein said *Citrus aurantium* extract is a flower extract.

8. Process for obtaining a fermented extract of aerial parts of *Citrus aurantium,* said process comprising the following steps:
a) extraction of aerial parts of Citrus aurantium with at least one alcoholic solvent and/or water;
b) filtration of the mixture obtained in a) in order to remove plant residues;
c) optionally, decolourization of the mixture obtained from step b);
d) removal of the extraction solvent and concentration of the resulting extract; and
e) fermentation of the extract obtained from *Citrus aurantium* by a yeast belonging to the species *Saccharomyces cerevisiae.*

9. Process according to Claim 8, wherein the fermentation step is carried out by means of a leaven prepared from the fermentation of a concentrated *Citrus aurantium* extract with the strain of *Saccharomyces cerevisiae.*

10. Cosmetic composition comprising an extract of *Citrus aurantium* according to any one of Claims 1 to 7.

11. Cosmetic use of the extract according to one of claims 1 to 7, for preventing/reducing skin ageing.

12. Cosmetic use of the extract according to one of claims 1 to 7 as an antioxidant active agent and/or to prevent/reduce oxidative stress and/or to detoxify the skin.
